# EUROPÄISCHE PATENTSCHRIFT

(11) **EP 3 362 848 B1**
(45) Veröffentlichungstag und Bekanntmachung des Hinweises auf die Patenterteilung: **10.04.2019**
(21) Anmeldenummer: 16784937.1
(22) Anmeldetag: 25.10.2016
(51) Int. Cl.: G02C 13/00, G01M 11/02

(54) **VERFAHREN UND VORRICHTUNG ZUR ÜBERPRÜFUNG DER BRECHKRAFTVERTEILUNG UND DER ZENTRIERUNG**
PROCESS AND APPARATUS FOR CHECKING THE POWER DISTRIBUTION AND CENTRING
PROCÉDÉ ET DISPOSITIF POUR VÉRIFIER LA DISTRIBUTION DE PUISSANCE ET LE CENTRAGE

(30) Priorität: 27.10.2015 DE 102015220931
(43) Veröffentlichungstag der Anmeldung: 22.08.2018
(73) Patentinhaber: Carl Zeiss Vision International GmbH, 73430 Aalen (DE)
(72) Erfinder: SCHÖN, Roland, 73433 Aalen (DE)
(74) Vertreter: Carl Zeiss AG - Patentabteilung
(86) Internationale Anmeldenummer: PCT/EP2016/075592
(87) Internationale Veröffentlichungsnummer: WO 2017/072085

(56) Entgegenhaltungen:
- US-A- 5 855 074
- US-A1- 2007 115 353
- US-A1- 2015 300 912

## Beschreibung

Die Erfindung ist in den Ansprüchen definiert. Unter Zentrierung versteht man in diesem Zusammenhang das korrekte Einarbeiten von Brillengläsern in eine Brillenfassung unter Berücksichtigung der Position und der korrekten Ausrichtung bzgl. der Pupillendistanz des Brillenträgers.

Ein Verfahren und eine Vorrichtung zum Überprüfen der Zentrierung von in eine Brillenfassung montierten Brillengläsern ist in der DE 10 2008 039 416 A1 beschrieben. Bei dem Verfahren werden zunächst die Positionen von Permanentmarkierungen in den Brillengläsern relativ zur Fassung ermittelt. Aus den ermittelten Positionen werden die Soll-Zentrierpunkte auf den Brillengläsern ermittelt. Mit Hilfe eines Videozentriergeräts werden Ist-Zentrierpunkte für den Brillenträger ermittelt. Ist- und Soll-Zentrierpunkte werden dann miteinander verglichen. Demnach wird bei diesem Verfahren die Lage der eingeschliffenen Brillengläser anhand der Permanentgravuren (ggf. automatisch) überprüft. Dieses Verfahren funktioniert nur bei Brillengläsern, auf denen solche Permanentgravuren auch vorhanden sind.

Die US 2007/0115353 A1 beschreibt eine Vorrichtung zum automatischen Erkennen von Permanentmarkierungen auf einem Brillenglas.

Die US 5,855,074 beschreibt eine Vorrichtung, um ein Brillenglas in einer Brillenfassung zu vermessen.

Die DE 10 2015 211 879 A1 beschreibt eine Vorrichtung und ein Verfahren zur Messung der Brechkraftverteilung eines Brillenglases einer in einer Messposition angeordneten Brille. Bei dem Verfahren wird die Brille in einer Messposition angeordnet. Es wird dann eine Teststruktur bereitgestellt. Die Abbildung der Teststruktur wird dann mit einem Abbildungsstrahlengang erfasst, der eines der Brillengläser der in der

Messposition angeordneten Brille durchsetzt. Die Brechkraftverteilung des Brillenglases wird dann aus den Koordinaten der Teststruktur und der erfassten Abbildung der Teststruktur und aus der räumlichen Lage des Brillenglases relativ zu der Teststruktur oder der Abbildung der Teststruktur bestimmt. Diese Methode ist auch geeignet, um Brillengläser ohne Permanentgravuren zu vermessen.

Der US 2015/300912 A1 entnimmt man ein Verfahren zur Bestimmung der Übereinstimmung einer tatsächlichen optischen Eigenschaft eines Brillenglases mit einer erwarteten optischen Eigenschaft auf der Basis von Testmustem. Das Verfahren umfasst folgende Schritte: a) Bestimmen eines Bildtestmuster eines ersten Testmusters durch das Brillenglas unter festgelegten optischen Bedingungen; b) Kombinieren des Bildtestmuster mit einem zweiten Testmuster unter Bildung eines Testtestmusters; c) Vergleichen das Testtestmuster mit wenigstens einem Referenztestmuster; und d) Ableiten der Übereinstimmung der tatsächlichen optischen Eigenschaft des Brillenglases mit der erwarteten optischen Eigenschaft auf Basis des Vergleichs. Die Ausrichtung des Brillenglases erfolgt beispielhaft anhand von Permanentgravuren.

Obwohl sich die oben beschriebenen Verfahren und Anordnungen dem Grunde nach bewährt haben, besteht das Bedürfnis nach einem einfachen Mittel zur Prozesskontrolle.

Die Aufgabe der vorliegenden Erfindung liegt daher darin, ein Verfahren und eine Vorrichtung bereitzustellen, welches sich zur Kontrolle des Einschleifprozesses eignet. Vorzugsweise soll eine universelle Einsetzbarkeit gewährleistet sein.

Diese Aufgabe wird durch Verfahren mit den Merkmalen der Patentansprüche 1 oder 14 und Vorrichtungen mit den Merkmalen der Patentansprüche 8 oder 15 gelöst. Vorteilhafte Ausführungen und Weiterbildungen der Erfindung sind Gegenstand der Unteransprüche.

Das erfindungsgemäße Verfahren zur Überprüfung der Zentrierung eines Brillenglases in einer Brillenfassung umfasst folgende Schritte:
- Anordnen des Brillenglases in einer Messposition,
- Bereitstellen einer Teststruktur,
- Erfassen einer Ist-Abbildung der Teststruktur für einen Abbildungsstrahl, der das in der Messposition angeordnete Brillenglas durchsetzt, und
   alternativ
- Vergleichen der erfassten Ist-Abbildung mit einer berechneten Soll-Abbildung der Teststruktur, welche sich für einen Abbildungsstrahl ergäbe, der ein in einer vorgegebenen Soll-Messposition angeordnetes Brillenglas mit einer vorgegebenen Soll-Brechkraftverteilung durchsetzen würde oder
- Vergleichen der erfassten Ist-Abbildung mit einer zu einer Soll-Abbildung der Teststruktur komplementären Soll-Komplement-Abbildung, wobei die Soll-Abbildung der Teststruktur diejenige Abbildung ist, welche sich für einen Abbildungsstrahl ergäbe, der ein in einer vorgegebenen Soll-Messposition angeordnetes Brillenglas mit einer vorgegebenen Soll-Brechkraftverteilung durchsetzen würde.

Das Verfahren setzt voraus, dass für die zu überprüfenden Eigenschaften Sollwerte vorliegen, d.h. es dient dazu im Rahmen einer Prozesskontrolle einen Soll-Ist-Vergleich vorzunehmen.

Es ist günstig, wenn die Teststruktur aus der vorgegebenen Soll-Abbildung berechnet wird. In diesem Fall kann nämlich eine vergleichsweise einfache geometrische Struktur, nämlich ein bekanntes Symbol oder ein häufig verwendetes Zeichen, wie z.B. ein kreisförmiger Ring, ein regelmäßiges Gitternetz oder dergleichen, als Soll-Abbildung vorgegeben werden, so dass Abweichungen der Ist-Abbildung von der Geometrie schnell feststellbar sind.

Das Berechnen der Teststruktur aus der vorgegebenen Soll-Abbildung kann z.B. mit einem Lichtstrahlverfolgungsverfahren erfolgen. Derartige Verfahren sind zur Berechnung optischer Elemente üblich, so dass es nur einer Anpassung, insbesondere einer Umkehrung des Rechenwegs, des ohnehin üblichen Equipments bedarf.

Es ist möglich, individuell angepasste Brillengläser im Hinblick auf Zentrierung vergleichsweise exakt zu überprüfen. In diesem Fall ist für jeden Einzelfall eine Soll-Brechkraftverteilung zu bestimmen, die der Brechkraftverteilung entspricht, die das zu überprüfende Brillenglas rechnerisch aufgrund der seiner Herstellung zugrunde liegenden Designdaten haben müsste.

Die Messposition ist erfindungsgemäß eine vorbestimmte Soll-Messposition der Brillenfassung hinsichtlich Ort und Orientierung.

Da es für die Genauigkeit des Vergleichs auf die Kenntnis des räumlichen Lagebezugs zwischen Ist-Messposition und Soll-Messposition der Brillenfassung hinsichtlich Ort und Orientierung ankommt, ist es erforderlich, sowohl die Ist-Messposition als auch die Soll-Messposition der Brillenfassung hinsichtlich Ort und Orientierung möglichst genau festlegen und/oder bestimmen zu können. Die Erfindung sieht daher vor, die vorgegebene Soll-Messposition der Brillenfassung hinsichtlich Ort und Orientierung aus den Koordinaten des Zentrierpunktes des zu überprüfenden Brillenglases zu berechnen.

Ein Vergleich der Ist-Abbildung und der Soll-Abbildung kann in verschiedenster Weise durchgeführt werden. Es ist z.B. möglich ein nicht veränderbares Soll-Abbildungsnormal vorzugeben, das in der Nähe der Überprüfungsapparatur angeordnet ist. Es ist auch möglich, Ist-Abbildung und Soll-Abbildung gar nicht zur Anzeige zu bringen, sondern mittels eines Computers einen rechnerischen Vergleich durchzuführen. Derartige Berechnungen können unter Umständen rechen- und damit zeitintensiv sein. Ein visueller Ansatz besteht darin, die Ist-Abbildung und die Soll-Abbildung gleichzeitig zur Anzeige zu bringen. Alternativ kann auch die Ist-Abbildung und die Soll-Komplement-Abbildung gleichzeitig angezeigt werden. Dies ermöglicht einer überprüfenden Person eine Entscheidung über die Qualität der Zentrierung des geprüften Brillenglases zu treffen.

Es ist möglich für die Durchführung des Vergleichs die Ist-Abbildung und die Soll-Abbildung nebeneinander anzuzeigen. Die Genauigkeit wird allerdings in der Regel gesteigert, wenn die Ist-Abbildung und die Soll-Abbildung einander überlagert angezeigt werden oder wenn die Ist-Abbildung und die Soll-Komplement-Abbildung einander überlagert angezeigt werden.

Die Genauigkeit der Durchführung des Vergleichs bei überlagerter Darstellung ist dann besonders groß, wenn die Ist-Abbildung und die Soll-Abbildung zur Deckung gebracht angezeigt werden oder wenn die Ist-Abbildung und die Soll-Komplement-Abbildung komplementär zueinander angezeigt werden.

Eine entsprechende erfindungsgemäße Vorrichtung zur Überprüfung der Zentrierung eines Brillenglases umfasst folgende Bestandteile:
- einen Halter zum Anordnen der Brillenfassung mit dem Brillenglas in einer Messposition,
- einer Anzeigeeinrichtung zum Anzeigen einer Teststruktur,
- einer Bilderfassungseinrichtung zum Erfassen einer Ist-Abbildung der Teststruktur für einen Abbildungsstrahl, der das in der Messposition angeordnete Brillenglas durchsetzt, und wahlweise
- eine Vergleichseinrichtung zum Vergleichen der erfassten Ist-Abbildung mit einer Soll-Abbildung der Teststruktur, wobei die Soll-Abbildung der Teststruktur diejenige Abbildung ist, die sich für einen Abbildungsstrahl ergäbe, der ein in einer vorgegebenen Soll-Messposition angeordnetes Brillenglas mit einer vorgegebenen Soll-Brechkraftverteilung durchsetzen würde oder zum Vergleichen der erfassten Ist-Abbildung mit einer zu einer Soll-Abbildung der Teststruktur komplementären Soll-Komplement-Abbildung, wobei die Soll-Abbildung der Teststruktur diejenige Abbildung ist, welche sich für einen Abbildungsstrahl ergäbe, der ein in einer vorgegebenen Soll-Messposition angeordnetes Brillenglas mit einer vorgegebenen Soll-Brechkraftverteilung durchsetzen würde;
   oder
- eine Anzeigeeinrichtung zum gleichzeitigen Anzeigen der erfassten Ist-Abbildung und einer Soll-Abbildung der Teststruktur, wobei die Soll-Abbildung der Teststruktur diejenige Abbildung ist, die sich für einen Abbildungsstrahl ergäbe, der ein in einer vorgegebenen Soll-Messposition angeordnetes Brillenglas mit einer vorgegebenen Soll-Brechkraftverteilung durchsetzen würde oder zum gleichzeitigen Anzeigen der erfassten Ist-Abbildung und einer zu einer Soll-Abbildung der Teststruktur komplementären Soll-Komplement-Abbildung, wobei die Soll-Abbildung der Teststruktur diejenige Abbildung ist, welche sich für einen Abbildungsstrahl ergäbe, der ein in einer vorgegebenen Soll-Messposition angeordnetes Brillenglas mit einer vorgegebenen Soll-Brechkraftverteilung durchsetzen würde.

Oben wurde ausgeführt, dass die Genauigkeit des Vergleichsergebnisses von den Ist- und Soll-Messpositionen abhängt. Wenn der Halter über Haltepunkte verfügt, deren Lage im Raum vorbekannt ist, kann die Brille mit dem zu überprüfenden Brillenglas vergleichsweise einfach in eine vorbestimmbare Position hinsichtlich Ort und Orientierung verbracht werden.

Es hat sich als günstig herausgestellt, wenn der Halter eine Kompensationseinrichtung zur Kompensation einer Vorneigung und/oder einer Seitenneigung der Brille mit dem zu überprüfenden Brillenglas aufweist. Diese kann z.B. in einer Kipp- und/oder Dreheinrichtung bestehen, um das Brillenglas oder die Brille mit dem Brillenglas entsprechend einer entsprechenden Vorgabe zu verkippen oder zu verdrehen. Es ist jedoch auch möglich, dass die Kompensationseinrichtung eine Halterung des Brillenglases oder der Brille mit dem Brillenglas ausschließlich in vomeigungs- und/oder einer seitenneigungskompensierenden Weise erlaubt.

Eine besonders vorteilhafte Ausgestaltungsform der Vorrichtung besteht darin, dass der Halter zum Halten einer Brille ausgebildet ist und dass der Halter eine Wechseleinrichtung für eine sequentielle Überprüfung der Zentrierung des rechten und linken Brillenglases der Brille aufweist. Die Wechseleinrichtung kann manuell bedienbar oder automatisiert ausgebildet sein. Die Wechseleinrichtung kann sich insbesondere dadurch auszeichnen, dass sie die jeweiligen Brillengläser in eine vorbestimmte oder vorbestimmbare Position verbringen kann.

Die erfindungsgemäße Vorrichtung kann auch eine Verschiebeeinrichtung zum relativen Verschieben des Halters und der Bilderfassungseinrichtung zueinander aufweisen, um z.B. eine verzerrte oder unscharfe Darstellung der Ist- und/oder Soll-Abbildungen zu korrigieren.

In entsprechender Weise kann die Vorrichtung zusätzlich oder alternativ eine Verschiebeeinrichtung zum relativen Verschieben des Halters und der Anzeigeeinrichtung aufweisen. Auch diese kann dazu dienen, verzerrte oder unscharfe Darstellungen der Ist- und/oder Soll-Abbildungen zu korrigieren.

Die Erfindung wird nachfolgend anhand der Zeichnungen näher beschrieben. Es zeigen:
- Figur 1: ein Ausführungsbeispiel einer Vorrichtung zur Überprüfung der Zentrierung eines Brillenglases;
- Figur 2a): einen Halter für eine Brille, welcher sich zum Einsatz bei der Vorrichtung nach der Figur 1 eignet,
- Figur 2b): den Halter nach der Figur 2a) mit von diesem gehaltener Brille;
- Figur 3: eine von dem Bildschirm der in Figur 1 gezeigten Vorrichtung angezeigte Teststruktur in Form einer Ellipse;
- Figur 4: die Anzeige des Computerbildschirms der in Figur 1 gezeigten Vorrichtung mit von der Kamera der in Figur 1 gezeigten Vorrichtung erfassten Ist-Abbildung und komplementärer Soll-Komplement-Abbildung.

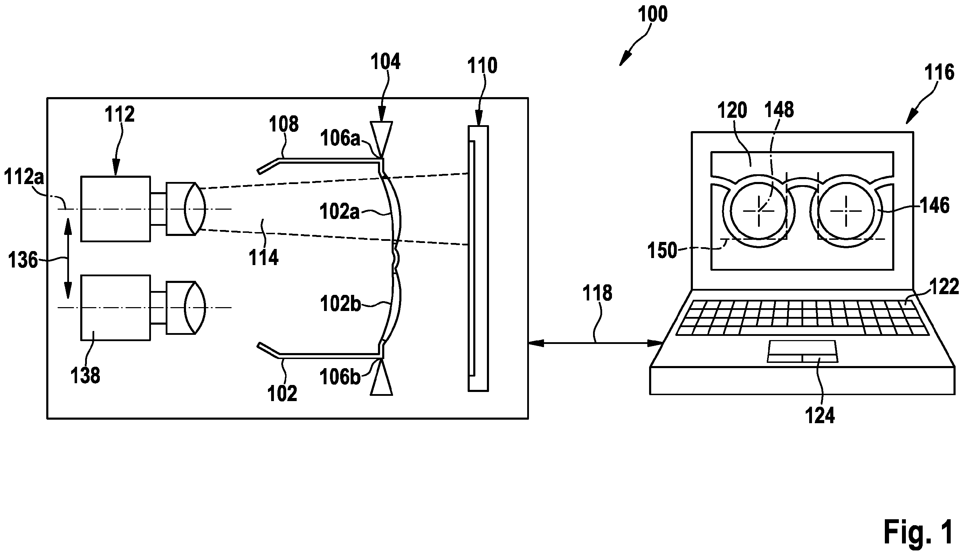

Die Figur 1 zeigt ein Ausführungsbeispiel einer erfindungsgemäßen Vorrichtung 100 zur Überprüfung der Zentrierung eines Brillenglases 102a. Die Vorrichtung 100 umfasst einen Halter 104 (skizziert mit Hilfe zweier Haltepunkte 106a, 106b), mit dem eine Brille 102 mit zwei in deren Fassung 108 montierten Brillengläser 102a, 102b in einer Messposition gehalten wird. Bestandteil der Vorrichtung 100 ist ein Bildschirm 110, der als Anzeigeeinrichtung zum Anzeigen einer Teststruktur dient. Als Bilderfassungseinrichtung ist eine Kamera 112 vorhanden. Diese dient der Erfassung einer Ist-Abbildung der auf dem Bildschirm 110 angezeigten Teststruktur, die sich für einen von dem Bildschirm ausgehenden

Abbildungsstrahl 114 ergibt, der das in der Messposition angeordnete in deren Fassung 108 montierte Brillenglas 102a durchsetzt und auf die Kamera 112 trifft.

Bestandteil der Vorrichtung 100 ist ein Computer 116. Der Computer 116 dient zur Datenaufnahme, Datenverarbeitung und der Steuerung von Bildschirm 110, Kamera 112 und ggf. Halter 104 (vgl. nachfolgende Beschreibung). Die entsprechende Datenverbindung vom Computer 116 zum Bildschirm 110, zur Kamera 112 und zum Halter 104 ist in der Zeichnung mit einem mit dem Bezugszeichen 118 gekennzeichneten Doppelpfeil gekennzeichnet. Zur Bedienung des Computers 116 ist eine Tastatur 122 mit Berührfeld 124 vorhanden.

Weiterer Bestandteil der Vorrichtung ist eine weitere Anzeigeeinrichtung, nämlich ein Bildschirm 120, der im vorliegenden Ausführungsbeispiel zum Computer 116 gehört. Dieser Bildschirm 120 dient dazu, die von der Kamera 112 erfasste Ist-Abbildung der Teststruktur sowie gleichzeitig eine Soll-Abbildung der Teststruktur oder eine zu der Soll-Abbildung komplementäre Soll-Komplement-Abbildung anzuzeigen. Eine Soll-Abbildung der Teststruktur ist hierbei diejenige Abbildung, die sich für einen Abbildungsstrahl ergäbe, der ein in einer vorgegebenen Soll-Messposition angeordnetes Brillenglas mit einer vorgegebenen Soll-Brechkraftverteilung durchsetzen würde.

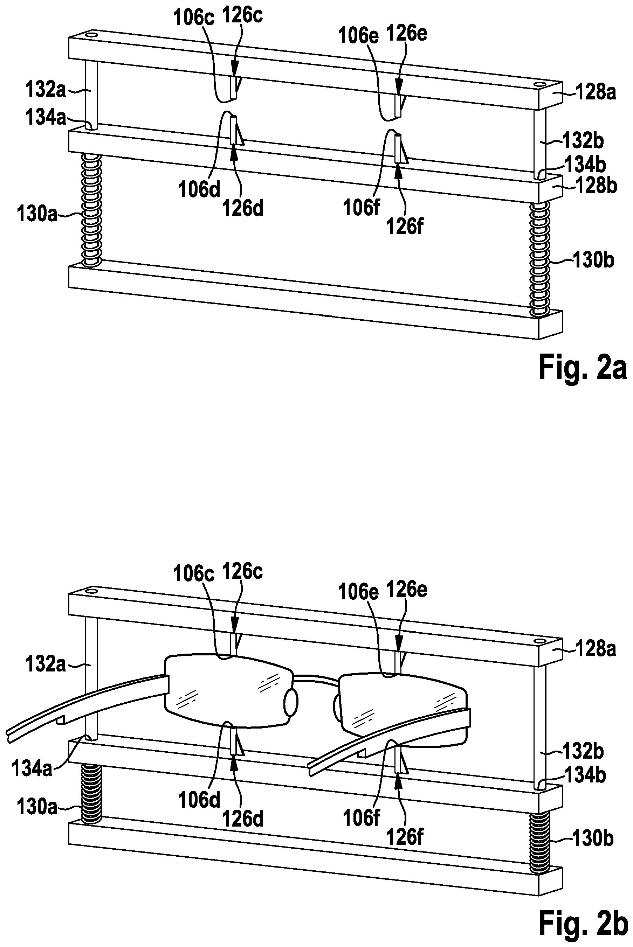

Ein Ausführungsbeispiel für einen Halter 104 ist in den Figuren 2a) und 2b) abgebildet. Die Figur 2a) zeigt den Halter 104 ohne Brille, die Figur 2b) zeigt den Halter nach der Figur 2a) mit von diesem gehaltener Brille. Der in den Figuren 2a) und 2b) gezeigte Halter 104 für die Brille 102 verfügt über Haltepunkte 106c, 106d, 106e, 106f deren Lage im Raum vollständig bekannt ist und der für eine eingespannte Brille 102 die Vorneigung der Brillengläser 102a, 102b kompensiert. Die Haltepunkte 106c, 106d, 106e, 106f sind die Kontaktpunkte von U-förmigen Aufnahmeelementen 126c, 126d, 126e, 126f, von denen jeweils zwei mit deren aufeinander zu gerichteten Öffnungen einander gegenüberliegend angeordnet sind. Die vier U-förmigen Aufnahmeelemente 126c, 126d, 126e, 126f werden von zwei parallel zueinander angeordneten Stangen 128a, 128b getragen. Die beiden Stangen 128a, 128b werden jeweils endseitig von zwei Tragestangen 132a, 132b gehalten. Die obere Stange 128a ist mit den beiden Tragestangen 132a, 132b fest verbunden. Die untere Stange 128b weist jeweils endseitig Öffnungen 134a, 134b auf, die von den Tragestangen 132a, 132b durchsetzt werden, so dass diese untere Stange 128b an den Tragestangen 132a, 132b parallel zur oberen Stange 128a verschiebbar geführt ist. Die untere Stange 128b kann gegen die Kraft zweier von den Tragestangen 132a, 132b durchsetzten Spiralfedern 130a, 130b verschoben werden.

Der in den Figuren 2a) und 2b) gezeigte Halter 104 erlaubt eine lagedefinierte Anordnung (bzgl. Ort und Orientierung) einer Brille im Raum ohne eine Möglichkeit oder ohne ein Erfordernis der Nachjustage. Es kann hilfreich sein, eine Justage per se vorzusehen. In einem solchen Fall muss der Halter in allen Raumrichtungen verschiebbar sowie verdreh- und verschwenkbar ausgebildet sein. Eine entsprechende elektronische Steuerung über den Computer 116 ist dabei von Vorteil. Weiter ist eine kameraunterstützte Lageerfassungseinrichtung hilfreich.

Um sowohl das linke, als auch das rechte Brillenglas sequentiell überprüfen zu können, ist entweder die Kamera 112 oder der Brillenhalter 104 senkrecht zur optischen Achse 112a der Kamera 112 verschiebbar (Richtungspfeil 136). Alternativ kann auch mit zwei Kameras 102, 138 gearbeitet werden. So können beide Brillengläser 102a, 102b gleichzeitig überprüft werden.

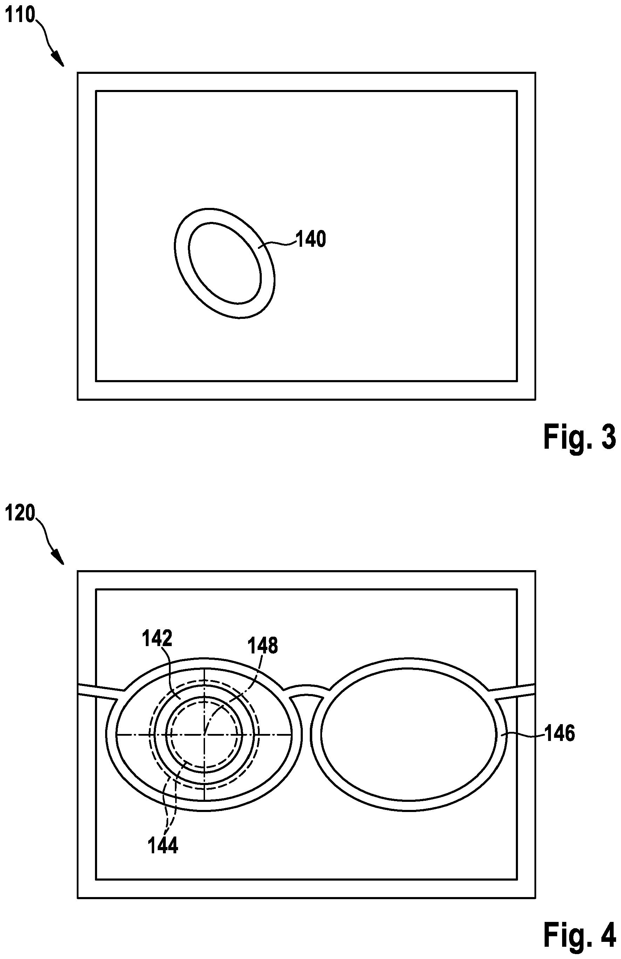

Auf dem Bildschirm 110 wird eine durch einen geeigneten Algorithmus, wie z.B. Raytracing berechnete Teststruktur, z.B. eine Ellipse 140, angezeigt (vgl. Figur 3). Die Teststruktur 140 ist so berechnet, dass sie von der Kamera 112 durch das Brillenglas 102a der Brille 104 betrachtet unverzerrt und abhängig von der Lage des optischen Zentrums des Brillenglases 102a an einer definierten Stelle des Bildsensors abgebildet wird. Diese Ist-Abbildung 142 wird auf dem Bildschirm 120 angezeigt (Figur 4).

Zur Berechnung der auf dem Bildschirm 110 anzuzeigenden Teststruktur 140 werden die Zentrierparameter und die refraktive Wirkung (Sphäre, Zylinder Achse, Prisma) des Brillenglases 102a benötigt, die der Vorrichtung 100 vor einer Messung übergeben werden müssen. Wird die berechnete Teststruktur 140 von der Kamera 112 unverzerrt, an der richtigen Position und mit der richtigen Größe abgebildet, so kann davon ausgegangen werden, dass das Brillenglas 102a mit der korrekten refraktiven Wirkung und der korrekten Zentrierung eingearbeitet wurde. Sind Abweichungen außerhalb einer festzulegenden Toleranz vom zu erwartenden Bild (welches der Soll-Abbildung der Teststruktur 140 entspricht) vorhanden, ist das Brillenglas 102a fehlerhaft.

Um eine Überprüfung der Verzerrung der Ist-Abbildung 142 zu erleichtern, wird auf dem Bildschirm 116 nicht nur die Ist-Abbildung 142 der Teststruktur 140 (und das von der Kamera 112 erfasste Bild 146 der Brille 102 angezeigt, sondern auch eine zu der Soll-Abbildung komplementäre Soll-Komplement-Abbildung 144 sowie ggf. weitere Hilfsstrukturen (siehe Figur 4). Als Hilfsstruktur zeigen die Figuren 1 und 4 ein Fadenkreuz. Die Figur 1 zeigt noch einen Teil eines Kastens 150.

Die Abbildung 142 der Teststruktur 140 durch das zu prüfende Brillenglas 102a wird in der Regel unscharf sein. Eine scharfe Abbildung kann erreicht werden, indem der Bildschirm 110 oder der Brillenhalter 104 parallel zur optischen Achse 112a des Kamerasystems 112, 138 verschoben wird. Aus der Position des Brillenhalters 104 bzw. des Bildschirms 110 kann so die Brechkraft des Brillenglases 102a (oder der Brillengläser 102a, 102b) ermittelt werden.

## Patentansprüche

1. Verfahren zur Überprüfung der Zentrierung eines Brillenglases (102a, 102b) in einer Brillenfassung (108) mit folgenden Schritten:
- Anordnen des in der Brillenfassung (108) montierten Brillenglases (102a, 102b) in einer Messposition,
- Bereitstellen einer Teststruktur (140),
- Erfassen einer Ist-Abbildung (142) der Teststruktur (140) für einen Abbildungsstrahl (114), der das in der Messposition angeordnete Brillenglas (102a, 102b) durchsetzt,
- Vergleichen der erfassten Ist-Abbildung (142) mit einer Soll-Abbildung der Teststruktur (140), welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste oder
- Vergleichen der erfassten Ist-Abbildung (142) mit einer zu einer Soll-Abbildung der Teststruktur (140) komplementären Soll-Komplement-Abbildung (144), wobei die Soll-Abbildung der Teststruktur (140) diejenige Abbildung ist, welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste
**dadurch gekennzeichnet, dass**
die Messposition eine vorbestimmte Soll-Messposition der Brillenfassung (108) hinsichtlich Ort und Orientierung ist.

2. Verfahren nach Anspruch 1, **dadurch gekennzeichnet, dass** die Teststruktur (140) aus der vorgegebenen Soll-Abbildung berechnet wird.

3. Verfahren nach Anspruch 2, **dadurch gekennzeichnet, dass** das Berechnen der Teststruktur (140) aus der vorgegebenen Soll-Abbildung mit einem Lichtstrahlverfolgungsverfahren erfolgt.

4. Verfahren nach einem der vorangegangenen Ansprüche, **dadurch gekennzeichnet, dass** die vorgegebene Soll-Messposition der Brillenfassung (108) hinsichtlich Ort und Orientierung aus den Koordinaten des Zentrierpunktes des Brillenglases (102a, 102b) in der Brillenfassung (108) berechnet wird.

5. Verfahren nach einem der vorangegangenen Ansprüche, **dadurch gekennzeichnet, dass**
- die Ist-Abbildung (142) und die Soll-Abbildung gleichzeitig angezeigt werden oder dass
- die Ist-Abbildung (142) und die Soll-Komplement-Abbildung (144) gleichzeitig angezeigt werden.

6. Verfahren nach Anspruch 5, **dadurch gekennzeichnet, dass**
- die Ist-Abbildung (142) und die Soll-Abbildung nebeneinander angezeigt werden oder dass
- die Ist-Abbildung (142) und die Soll-Abbildung einander überlagert angezeigt werden oder dass
- die Ist-Abbildung (142) und die Soll-Komplement-Abbildung (144) einander überlagert angezeigt werden.

7. Verfahren nach einem der Ansprüche 5 oder 6, **dadurch gekennzeichnet, dass**
- die Ist-Abbildung (142) und die Soll-Abbildung zur Deckung gebracht angezeigt werden oder dass
- die Ist-Abbildung (142) und die Soll-Komplement-Abbildung (144) komplementär zueinander angezeigt werden.

8. Vorrichtung (100) zur Überprüfung der Zentrierung eines Brillenglases (102a, 102b) in einer Brillenfassung (108) nach dem Verfahren von Anspruch 1, umfassend
- einen Halter (104) zum Anordnen des Brillenglases (102a, 102b) in einer Messposition,
- eine Anzeigeeinrichtung (110) zum Anzeigen einer Teststruktur (140),
- eine Bilderfassungseinrichtung (112, 138) zum Erfassen einer Ist-Abbildung (142) der Teststruktur (140) für einen Abbildungsstrahl (114), der das in der Messposition angeordnete Brillenglas (102a, 102b) durchsetzt,
- eine Vergleichseinrichtung zum Vergleichen der erfassten Ist-Abbildung (142) mit einer Soll-Abbildung der Teststruktur (140), wobei die Soll-Abbildung der Teststruktur (140) diejenige Abbildung ist, die sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste, oder zum Vergleichen der erfassten Ist-Abbildung (142) mit einer zu einer Soll-Abbildung der Teststruktur (140) komplementären Soll-Komplement-Abbildung, wobei die Soll-Abbildung der Teststruktur (140) diejenige Abbildung ist, welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste;
oder
- eine Anzeigeeinrichtung (120) zum gleichzeitigen Anzeigen der erfassten Ist-Abbildung (142) und einer Soll-Abbildung der Teststruktur (140), wobei die Soll-Abbildung der Teststruktur (140) diejenige Abbildung ist, die sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste, oder zum gleichzeitigen Anzeigen der erfassten Ist-Abbildung (142) und einer zu einer Soll-Abbildung der Teststruktur (140) komplementären Soll-Komplement-Abbildung (144), wobei die Soll-Abbildung der Teststruktur (140) diejenige Abbildung ist, welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste **dadurch gekennzeichnet, dass**
der Halter (104) ausgebildet ist, die Brillenfassung (108) hinsichtlich Ort und Orientierung in einer vorbestimmten Soll-Messposition der Brillenfassung (108) zu halten.

9. Vorrichtung (100) nach Anspruch 8, **dadurch gekennzeichnet, dass** der Halter (104) über Haltepunkte (106a, 106b, 106c, 106d, 106e, 106f) verfügt, deren Lage im Raum vorbekannt ist.

10. Vorrichtung (100) nach Anspruch 9, **dadurch gekennzeichnet, dass** der Halter (104) eine Kompensationseinrichtung zur Kompensation einer Vorneigung und/oder einer Seitenneigung aufweist.

11. Vorrichtung (100) nach einem der Ansprüche 8 bis 10, **dadurch gekennzeichnet, dass** der Halter (104) zum Halten einer Brille (102) ausgebildet ist und dass der Halter (104) eine Wechseleinrichtung (138) für eine sequentielle Überprüfung der Brechkraftverteilung und/oder der Zentrierung des rechten und linken Brillenglases (102a, 102b) der Brille (102) aufweist.

12. Vorrichtung (100) nach einem der Ansprüche 8 bis 11, **gekennzeichnet durch** eine Verschiebeeinrichtung zum relativen Verschieben des Halters (104) und der Bilderfassungseinrichtung (112, 138) zueinander.

13. Vorrichtung nach einem der Ansprüche 8 bis 12, **gekennzeichnet durch** eine Verschiebeeinrichtung zum relativen Verschieben des Halters (104) und der Anzeigeeinrichtung (110) zum Anzeigen der Teststruktur (140).

14. Verfahren nach einem der Ansprüche 1 bis 7, **dadurch gekennzeichnet, dass** die vorgegebene Soll-Messposition der Brillenfassung (108) aus den Koordinaten des Zentrierpunktes des Brillenglases (102a, 102b) in der Brillenfassung (108) berechnet wird.

15. Vorrichtung (100) nach einem der Ansprüche 8 bis 13, umfassend
- einen Halter (104) zum Anordnen des in der Brillenfassung (108) montierten Brillenglases (102a, 102b) in einer Messposition,
- eine Anzeigeeinrichtung (110) zum Anzeigen einer Teststruktur (140),
- eine Bilderfassungseinrichtung (112, 138) zum Erfassen einer Ist-Abbildung (142) der Teststruktur (140) für einen Abbildungsstrahl (114), der das in der Messposition angeordnete Brillenglas (102a, 102b) durchsetzt,
- eine Vergleichseinrichtung zum Vergleichen der erfassten Ist-Abbildung (142) mit einer Soll-Abbildung der Teststruktur (140), wobei die Soll-Abbildung der Teststruktur (140) diejenige Abbildung ist, die sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste, oder zum Vergleichen der erfassten Ist-Abbildung (142) mit einer zu einer Soll-Abbildung der Teststruktur (140) komplementären Soll-Komplement-Abbildung, wobei die Soll-Abbildung der Teststruktur (140) diejenige Abbildung ist, welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste,
oder
- eine Anzeigeeinrichtung (120) zum gleichzeitigen Anzeigen der erfassten Ist-Abbildung (142) und einer Soll-Abbildung der Teststruktur (140), wobei die Soll-Abbildung der Teststruktur (140) diejenige Abbildung ist, die sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste, oder zum gleichzeitigen Anzeigen der erfassten Ist-Abbildung (142) und einer zu einer Soll-Abbildung der Teststruktur (140) komplementären Soll-Komplement-Abbildung (144), wobei die Soll-Abbildung der Teststruktur (140) diejenige Abbildung ist, welche sich für einen Abbildungsstrahl ergäbe, der ein in einer vorgegebenen Soll-Messposition angeordnetes Vergleichs-Brillenglas mit einer vorgegebenen Soll-Brechkraftverteilung durchsetzen würde, wobei die Soll-Brechkraftverteilung die Brechkraftverteilung ist, die das zu überprüfende Brillenglas (102a, 102b) rechnerisch aufgrund der seiner Herstellung zugrunde liegenden Designdaten haben müsste,
**dadurch gekennzeichnet, dass**
der Halter (104) ausgebildet ist, die Brillenfassung (108) hinsichtlich Ort und Orientierung in einer vorbestimmten Soll-Messposition der Brillenfassung (108) zu halten, wobei die vorgegebene Soll-Messposition der Brillenfassung (108) aus den Koordinaten des Zentrierpunktes des Brillenglases (102a, 102b) in der Brillenfassung (108) berechnet wird.

## Claims

1. Method for checking the centering of a spectacle lens (102a, 102b) in a spectacle frame (108) comprising the following steps:
- arranging the spectacle lens (102a, 102b) mounted in the spectacle frame (108) in a measurement position,
- providing a test structure (140),
- capturing an actual imaging (142) of the test structure (140) for an imaging beam (114) which passes through the spectacle lens (102a, 102b) arranged in the measurement position,
- comparing the captured actual imaging (142) with a setpoint imaging of the test structure (140) which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based, or
- comparing the captured actual imaging (142) with a setpoint complement imaging (144) complementary to a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based,
**characterized in that**
the measurement position is a predetermined setpoint measurement position of the spectacle frame (108) with regard to location and orientation.

2. Method according to Claim 1, **characterized in that** the test structure (140) is calculated from the predefined setpoint imaging.

3. Method according to Claim 2, **characterized in that** the test structure (140) is calculated from the predefined setpoint imaging by a light ray tracing method.

4. Method according to any of the preceding claims, **characterized in that** the predefined setpoint measurement position of the spectacle frame (108) with regard to location and orientation is calculated from the coordinates of the centering point of the spectacle lens (102a, 102b) in the spectacle frame (108).

5. Method according to any of the preceding claims, **characterized in that**
- the actual imaging (142) and the setpoint imaging are displayed simultaneously or **in that**
- the actual imaging (142) and the setpoint complement imaging (144) are displayed simultaneously.

6. Method according to Claim 5, **characterized in that**
- the actual imaging (142) and the setpoint imaging are displayed alongside one another or **in that**
- the actual imaging (142) and the setpoint imaging are displayed in a manner superimposed on one another or **in that**
- the actual imaging (142) and the setpoint complement imaging (144) are displayed in a manner superimposed on one another.

7. Method according to either of Claims 5 and 6, **characterized in that**
- the actual imaging (142) and the setpoint imaging are displayed in a manner brought to congruence or **in that**
- the actual imaging (142) and the setpoint complement imaging (144) are displayed complementarily with respect to one another.

8. Device (100) for checking the centering of a spectacle lens (102a, 102b) in a spectacle frame (108) according to the method of Claim 1, comprising
- a holder (104) for arranging the spectacle lens (102a, 102b) in a measurement position,
- a display unit (110) for displaying a test structure (140),
- an image capture unit (112, 138) for capturing an actual imaging (142) of the test structure (140) for an imaging beam (114) which passes through the spectacle lens (102a, 102b) arranged in the measurement position,
- a comparison unit for comparing the captured actual imaging (142) with a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based, or for comparing the captured actual imaging (142) with a setpoint complement imaging complementary to a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based;
or
- a display unit (120) for simultaneously displaying the captured actual imaging (142) and a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based, or for simultaneously displaying the captured actual imaging (142) and a setpoint complement imaging (144) complementary to a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based,
**characterized in that**
the holder (104) is configured to hold the spectacle frame (108) with regard to location and orientation in a predetermined setpoint measurement position of the spectacle frame (108).

9. Device (100) according to Claim 8, **characterized in that** the holder (104) has holding points (106a, 106b, 106c, 106d, 106e, 106f), the position of which in space is previously known.

10. Device (100) according to Claim 9, **characterized in that** the holder (104) has a compensation unit for compensating for a forward inclination and/or a lateral inclination.

11. Device (100) according to any of Claims 8 to 10, **characterized in that** the holder (104) is configured for holding spectacles (102), and **in that** the holder (104) comprises an interchange unit (138) for sequentially checking the refractive power distribution and/or the centering of the right and left spectacle lenses (102a, 102b) of the spectacles (102).

12. Device (100) according to any of Claims 8 to 11, **characterized by** a displacement unit for relatively displacing the holder (104) and the image capture unit (112, 138) with respect to one another.

13. Device according to any of Claims 8 to 12, **characterized by** a displacement unit for relatively displacing the holder (104) and the display unit (110) for displaying the test structure (140).

14. Method according to any of Claims 1 to 7, **characterized in that** the predefined setpoint measurement position of the spectacle frame (108) is calculated from the coordinates of the centering point of the spectacle lens (102a, 102b) in the spectacle frame (108).

15. Device (100) according to any of Claims 8 to 13, comprising
- a holder (104) for arranging the spectacle lens (102a, 102b) mounted in the spectacle frame (108) in a measurement position,
- a display unit (110) for displaying a test structure (140),
- an image capture unit (112, 138) for capturing an actual imaging (142) of the test structure (140) for an imaging beam (114) which passes through the spectacle lens (102a, 102b) arranged in the measurement position,
- a comparison unit for comparing the captured actual imaging (142) with a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based, or for comparing the captured actual imaging (142) with a setpoint complement imaging complementary to a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based;
or
- a display unit (120) for simultaneously displaying the captured actual imaging (142) and a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based, or for simultaneously displaying the captured actual imaging (142) and a setpoint complement imaging (144) complementary to a setpoint imaging of the test structure (140), wherein the setpoint imaging of the test structure (140) is that imaging which would arise for an imaging beam which would pass through a comparative spectacle lens arranged in a predefined setpoint measurement position with a predefined setpoint refractive power distribution, wherein the setpoint refractive power distribution is the refractive power distribution which the spectacle lens (102a, 102b) to be checked should have computationally on account of the design data on which its production is based,
**characterized in that**
the holder (104) is configured to hold the spectacle frame (108) with regard to location and orientation in a predetermined setpoint measurement position of the spectacle frame (108), wherein the predefined setpoint measurement position of the spectacle frame (108) is calculated from the coordinates of the centering point of the spectacle lens (102a, 102b) in the spectacle frame (108) .

## Revendications

1. Procédé de contrôle du centrage d'un verre de lunettes (102a, 102b) dans une monture de lunettes (108) avec les étapes suivantes :
- placement, dans une position de mesure, du verre de lunettes (102a, 102b) monté dans la monture de lunettes (108),
- fourniture d'une structure de test (140),
- détection d'une image effective (142) de la structure de test (140) pour un faisceau d'image (114) qui pénètre dans le verre de lunettes (102a, 102b) disposé dans la position de mesure,
- comparaison de l'image effective (142) détectée avec une image de consigne de la structure de test (140) qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication, ou
- comparaison de l'image effective (142) détectée avec une image complémentaire de consigne (144) complémentaire à une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication,
**caractérisé en ce que**
la position de mesure est une position de mesure de consigne prédéfinie de la monture de lunettes (108) en ce qui concerne le lieu et l'orientation.

2. Procédé selon la revendication 1, **caractérisé en ce que** la structure de test (140) est calculée à partir de l'image de consigne prédéfinie.

3. Procédé selon la revendication 2, **caractérisé en ce que** le calcul de la structure de test (140) s'effectue à partir de l'image de consigne prédéfinie avec un procédé de suivi de faisceau lumineux.

4. Procédé selon l'une des revendications précédentes, **caractérisé en ce que** la position de mesure de consigne prédéfinie de la monture de lunettes (108) en ce qui concerne le lieu et l'orientation est calculée à partir des coordonnées du point de centrage du verre de lunettes (102a, 102b) dans la monture de lunettes (108) .

5. Procédé selon l'une des revendications précédentes, **caractérisé en ce que**
- l'image effective (142) et l'image de consigne sont affichées simultanément ou **en ce que**
- l'image effective (142) et l'image complémentaire de consigne (144) sont affichées simultanément.

6. Procédé selon la revendication 5, **caractérisé en ce que**
- l'image effective (142) et l'image de consigne sont affichées de façon juxtaposée ou **en ce que**
- l'image effective (142) et l'image de consigne sont affichées de façon superposée l'une à l'autre ou **en ce que**
- l'image effective (142) et l'image complémentaire de consigne (144) sont affichées de façon superposée l'une à l'autre.

7. Procédé selon l'une des revendications 5 ou 6, **caractérisé en ce que**
- l'image effective (142) et l'image de consigne sont affichées alors qu'elles ont été amenées à se recouvrir l'une l'autre ou **en ce que**
- l'image effective (142) et l'image complémentaire de consigne (144) sont affichées de façon complémentaire l'une à l'autre.

8. Dispositif (100) de contrôle du centrage d'un verre de lunettes (102a, 102b) dans une monture de lunettes (108) selon le procédé selon la revendication 1, comprenant
- un élément de retenue (104) destiné à placer le verre de lunettes (102a, 102b) dans une position de mesure,
- un système d'affichage (110) destiné à l'affichage d'une structure de test (140),
- un système de détection d'image (112, 138) destiné à la détection d'une image effective (142) de la structure de test (140) pour un faisceau d'image (114) qui pénètre dans le verre de lunettes (102a, 102b) disposé dans la position de mesure,
- un système de comparaison destiné à la comparaison de l'image effective (142) détectée avec une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication, ou destiné à la comparaison de l'image effective (142) détectée avec une image complémentaire de consigne complémentaire à une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication ;
ou
- un système d'affichage (120) destiné à l'affichage simultané de l'image effective (142) détectée et d'une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication, ou destiné à l'affichage simultané de l'image effective (142) détectée et d'une image complémentaire de consigne (144) complémentaire à une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication,
**caractérisé en ce que**
l'élément de retenue (104) est constitué pour retenir la monture de lunettes (108) dans une position de mesure de consigne prédéfinie de la monture de lunettes (108) en ce qui concerne le lieu et l'orientation.

9. Dispositif (100) selon la revendication 8, **caractérisé en ce que** l'élément de retenue (104) dispose de points de retenue (106a, 106b, 106c, 106d, 106e, 106f) dont la position dans l'espace est connue d'avance.

10. Dispositif (100) selon la revendication 9, **caractérisé en ce que** l'élément de retenue (104) comporte un système de compensation destiné à compenser une inclinaison frontale et/ou une inclinaison latérale.

11. Dispositif (100) selon l'une des revendications 8 à 10, **caractérisé en ce que** l'élément de retenue (104) est constitué pour la retenue d'une paire de lunettes (102) et **en ce que** l'élément de retenue (104) est un système de changement (138) pour un contrôle séquentiel de la répartition du pouvoir de réfraction et/ou du centrage du verre de lunette droit et gauche (102a, 102b) de la paire de lunettes (102).

12. Dispositif (100) selon l'une des revendications 8 à 11, **caractérisé par** un système de déplacement destiné au déplacement relatif de l'élément de retenue (104) et du système de détection d'image (112, 138) l'un par rapport à l'autre.

13. Dispositif selon l'une des revendications 8 à 12, **caractérisé par** un système de déplacement destiné au déplacement relatif de l'élément de retenue (104) et du système d'affichage (110) destiné à l'affichage de la structure de test (140).

14. Procédé selon l'une des revendications 1 à 7, **caractérisé en ce que** la position de mesure de consigne prédéfinie de la monture de lunettes (108) est calculée à partir des coordonnées du point de centrage du verre de lunettes (102a, 102b) dans la monture de lunettes (108) .

15. Dispositif (100) selon l'une des revendications 8 à 13, comprenant
- un élément de retenue (104) destiné à placer le verre de lunettes (102a, 102b) monté dans la monture de lunettes (108) dans une position de mesure,
- un système d'affichage (110) destiné à l'affichage d'une structure de test (140),
- un système de détection d'image (112, 138) destiné à la détection d'une image effective (142) de la structure de test (140) pour un faisceau d'image (114) qui pénètre dans le verre de lunettes (102a, 102b) disposé dans la position de mesure,
- un système de comparaison destiné à la comparaison de l'image effective (142) détectée avec une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication, ou destiné à la comparaison de l'image effective (142) détectée avec une image complémentaire de consigne complémentaire à une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication ;
ou
- un système d'affichage (120) destiné à l'affichage simultané de l'image effective (142) détectée et d'une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication, ou destiné à l'affichage simultané de l'image effective (142) détectée et d'une image complémentaire de consigne (144) complémentaire à une image de consigne de la structure de test (140), l'image de consigne de la structure de test (140) étant l'image qui serait obtenue pour un faisceau d'image qui pénétrerait dans un verre de lunettes de comparaison disposé dans une position de mesure de consigne prédéfinie et ayant une répartition du pouvoir de réfraction de consigne prédéfinie, la répartition du pouvoir de réfraction de consigne étant la répartition du pouvoir de réfraction que devrait avoir le verre de lunettes (102a, 102b) à contrôler selon le calcul basé sur les données de conception qui sous-tendent sa fabrication,
**caractérisé en ce que**
l'élément de retenue (104) est constitué pour retenir la monture de lunettes (108) dans une position de mesure de consigne prédéfinie de la monture de lunettes (108) en ce qui concerne le lieu et l'orientation, la position de mesure de consigne prédéfinie de la monture de lunettes (108) étant calculée à partir des coordonnées du point de centrage du verre de lunettes (102a, 102b) dans la monture de lunettes (108).
